# EUROPÄISCHE PATENTSCHRIFT

(11) **EP 2 426 116 B1**
(45) Veröffentlichungstag und Bekanntmachung des Hinweises auf die Patenterteilung: **26.06.2013**
(21) Anmeldenummer: 10174550.3
(22) Anmeldetag: 30.08.2010
(51) Int. Cl.: C07D 333/20

(54) **Verfahren zur Herstellung von (S)-3-N-Methylamino-1-(2-thienyl)-1-propanol**
Method for producing (S)-3-N-Methylamino-1-(2-thienyl)-1-propanol
Procédé de fabrication de (S)-3-N-méthylamino-1-(2-thiényl)-1-propanol

(43) Veröffentlichungstag der Anmeldung: 07.03.2012
(73) Patentinhaber: Saltigo GmbH, 40764 Langenfeld (DE)
(72) Erfinder: Kreis, Michael, 51373, Leverkusen (DE); Hieronymi, Antje, 50733, Köln (DE); Giffels, Guido, 53177, Bonn (DE); Krebs, Oliver, 40764, Langenfeld (DE)
(74) Vertreter: Wichmann, Birgid

(56) Entgegenhaltungen:
- EP-A1- 1 346 972
- EP-A1- 1 346 977
- WO-A2-2004/031168
- WO-A2-2010/003942
- CN-A- 101 104 614
- US-A1- 2003 207 877

## Beschreibung

Die vorliegende Erfindung betrifft ein Verfahren zur Herstellung von (S)-3-N-Methylamino-1-(2-thienyl)-1-propanol und dessen Salze.

(S)-3-N-Methylamino-1-(2-thienyl)-1-propanol stellt ein wichtiges Zwischenprodukt bei der Synthese der bioaktiven Substanz (S)-N-Methyl-3-(1-naphtyloxy)-3-(2-thienyl)propanamin Hydrochlorid (Duloxetine^{®}) dar. (S)-N-Methyl-3-(1-naphtyloxy)-3-(2-thienyl)propanamin Hydrochlorid wirkt als Serotoninwiederaufnahme Inhibitor und wird als Antidepressivum sowie zur Behandlung von Inkontizenz eingesetzt (Huiling et al. Chirality, 2000, 12, 26-29 und Sorbera, Drugs ofthe Future, 2000, 25, 907-916).

WO 2004031168 beschreibt die Herstellung von (*S*)-3-N-Methylamino-1-(2-thienyl)-1-propanol durch asymmetrische Transferhydrierung von (S)-3-N-Methylamino-1-(2-thienyl)-1-propanon mit (1*S*,2*R*)-(-)-*cis*-1-Amino-2-indanol als chiralem Liganden und (p-Cymol)Ruthenium(II)-Dimer als Katalysatorvorläufer. Nachteilig an diesem Verfahren ist, dass lediglich geringe Enantiomerenüberschüsse erreicht werden und daher eine diastereoselektive Kristallisation mit einer teuren chiralen Säure als zusätzlichen Reaktionsschritt notwendig wird.

Aus EP 1510517 A, Angew. Chem. Int. Ed., 2005, 44, 1687 und WO 2004020389 ist bekannt, dass (*S*)-3-N-Methylamino-1-(2-thienyl)-1-propanol durch asymmetrische Hydrierung von 3-N-Methylamino-1-(2-thienyl)-1-propanon unter Verwendung chiraler bidentaler Phosphinliganden hergestellt werden kann. WO 2006087166 beschreibt ein Verfahren zur Herstellung von (*S*)-3-N-Methylamino-1-(2-thienyl)-1-propanol Sulfonsäuresalzen durch asymmetrische Hydrierung von 3-N-Methylamino-1-(2-thienyl)-1-propanon Sulfonsäuresalzen ebenfalls unter Verwendung chiraler bidentaler Phosphinliganden. Den Verfahren ist gemeinsam, dass sie in technischen Prozessen nicht effizient sind, da teuere Phophinliganden eingesetzt werden und verfahrenstechnisch aufwendige Anlagen benötigt werden, da mit Wasserstoff unter hohen Drücken hydriert wird.

Ein weiteres Verfahren zur Herstellung von (*S*)-3-N-Methylamino-1-(2-thienyl)-1-propanol durch enzymatisch katalysierte Reduktion von 3-N-Methylamino-1-(2-thienyl)-1-propanon ist aus WO 2004065376 bekannt. Die eingesetzten Mikroorganismen lassen sich nur aufwendig kultivieren, so dass das Verfahren in technischen Prozessen nicht effizient durchführbar ist.

WO 2004011452 beschreibt, dass die asymmetrische Reduktion von 3-N-Methylamino-1-(2-thienyl)-1-propanon zu (*S*)-3-N-Methylamino-1-(2-thienyl)-1-propanol unter Verwendung von Rutheniumkatalysatoren und chiraler, bidentaler Phosphinliganden sowie Diaminliganden ebenfalls unter Transferhydrierung durchgeführt werden kann. Nachteilig an diesem Verfahren ist, dass die erzielten enantiomeren Reinheiten und Ausbeuten unzureichend sind.

Aus EP 1340746 ist ein Verfahren zur Herstellung von 3-N-Methylamino-1-(2-thienyl)-1-propanon durch asymmetrische Transferhydrierung von β-Ketosäureestern zu β-Hydroxysäureestern, insbesondere zum (*S*)-Methyl-3-hydroxy-3-(2-thienyl)-propanoat, und weiterer Reduktion zum 3-N-Methylamino-1-(2-thienyl)-1-propanon bekannt. Da teure und sicherheitstechnisch bedenkliche Reduktionsmittels, wie z.B. Lithiumaluminiumhydrid, verwendet werden müssen, ist dieses Verfahren in technischen Prozessen nicht effizient durchführbar.

Es bestand daher weiterhin das Bedürfnis nach einem Verfahren zur Herstellung von (*S*)-3-N-Methylamino-1-(2-thienyl)-1-propanol mit dem die Nachteile des Standes der Technik überwunden werden können und (*S*)-3-N-Methylamino-1-(2-thienyl)-1-propanol in hohen enantiomeren Reinheiten und guten Ausbeuten hergestellt werden kann.

Überraschenderweise wurde ein Verfahren, basierend auf einer asymmetrischen Transferhydrierung gefunden, mit dem die Nachteile des Standes der Technik überwunden werden konnten und (*S*)-3-N-Methylamino-1-(2-thienyl)-1-propanol ökonomisch effizient, in hohen enantiomeren Reinheiten und guten Ausbeuten in technischen Prozessen hergestellt werden kann.

Gegenstand der Erfindung ist daher ein Verfahren zur Herstellung von (*S*)-3-N-Methylamino-1-(2-thienyl)-1-propanol und dessen Salze in dem 3-N-Methylamino-l-(2-thienyl)-1-propanon - Hydrochlorid oder Methansulfat in Gegenwart p-Cymoldichlororutherium-Dimer und 5,5-N-p Tolnolyulfonyl-1,2-diphenylethylendiamin
in Gegenwart mindestens eines Amins und
in Gegenwart von Ameisensäure oder/und Formiaten oder Mischungen davon
umgesetzt wird.

Amine stehen im Sinne der Erfindung für cyklische, verzweigte oder unverzweigte Mono-, Di-, oder tertiäre Alkylamine oder Mono-, Di-, oder tertiäre Arylamine oder Gemische dieser Amine. Besiepielsweise stehen Amine für Methylamin, Ethylamin, Propylamin, iso-Propylamin, Triethylamin, Trimethylamin, Triphenylamin Tripropylamin, Tripentylamin, n-Butylamin, isoButylamin, tert.-Butylamin, cyclo-Hexylamin, cyclo-Octylamin, Pentylamin oder Octylamin. Bevorzugt steht Amin für ein tertiäres Alkyl- oder Arylamin, wie z.B. Triethylamin, Trimethylamin, Triphenylamin Tripropylamin, Tripentylamin oder tert.-Butylamin. Besonders bevorzugt steht Amin für ein tertiäres Alkylamin. Noch weiter bevorzugt steht Amin für Triethylamin.

Der Rahmen der Erfindung erfasst alle oben stehenden und im Folgenden aufgeführten allgemeinen oder in Vorzugsbereichen genannten Restedefinitionen, Parameter und Erläuterungen untereinander, also auch zwischen den jeweiligen Bereichen und Vorzugsbereichen in beliebiger Kombination.

Das erfindungsgemäße Verfahren kann in Gegenwart oder Abwesenheit von Lösungsmitteln durchgeführt werden. Bevorzugt wird das Verfahren in Gegenwart von organischem Lösungsmittel durchgeführt.

Als Lösungsmittel zur Durchführung des erfindungsgemäßen Verfahrens eignen sich insbesondere Amide wie z.B. Dimethylformamid, N-Methylpyrrolidinon, aliphatische oder aromatische gegebenenfalls halogenierte Lösungsmittel mit bis zu 16 Kohlenstoffatomen wie z.B. Toluol, o-, m-, p-Xylol, Chloroform, Dichlormethan, Chlorbenzol, die isomeren Dichlorbenzole, Fluorbenzol, Nitrile wie z.B. Acetonitril, Benzonitril, Dimethylsulfoxid oder Alkohole, wie z.B. Methanol, Ethanol, Propanol oder Butanol oder Mischungen der genannten Lösungsmittel.

Bevorzugte Lösungsmittel sind Amide, Ntrile oder Alkohole oder Mischungen dieser Lösungsmittel. Besonders bevorzugt werden als Lösungsmittel Acetonitril, Dimethylformamid, Dimethylacetamid, N-Methylpyrrolidinon oder Methanol oder Mischungen dieser Lösungsmittel eingesetzt.

Bevorzugt werden Mischungen von Ameisensäure mit Aminen eingesetzt. Auf diese Weise bilden sich zumindest teilweise die entsprechenden Ammoniumformiate der, die natürlich analog eingesetzt werden können.

Das molare Verhältnis von Ameisensäure zu tertiärem Amin kann beispielsweise und vorzugsweise 10:1 bis 1:10 betragen, besonders bevorzugt 3:1 bis 0,8:1 betragen.

Das molare Verhältnis an Ameisensäure bezogen auf eingesetztes Substrat beträgt vorzugsweise 10:1 bis 1:1.

Das erfindungsgemäße Verfahren kann beispielsweise bei Temperaturen zwischen 0 bis 100 °C, bevorzugt zwischen 20°C bis 80 °C, besonders bevorzugt bei Temperaturen zwischen 30°C bis 60 °C durchgeführt werden.

Im Allgemeinen können die Reaktionszeiten im erfindungsgemäßen Verfahren beliebig gewählt werden. Bevorzugt liegen die Reaktionszeiten zwischen 0,1 h und 72 h, besonders bevorzugt zwischen 1 h und 48 h.

Die eingesetzte molare Menge an Ruthenium im erfindungsgemäßen Verfahren kann beispielsweise 0,05 bis 10 mol-% bezogen auf die eingesetzte Menge an 3-N-Methylamino-1-(2-thienyl)-1-propanon- Hydrochlorid- oder Methansulfat betragen. Bevorzugt beträgt die im erfindungsgemäßen Verfahren eingesetzte Menge an Ruthenium 0,05 bis 5 mol-% besonders bevorzugt 0,05 bis 1 mol-%.

Das molare Verhältnis von Verbindungen der Formel (I) zu Verbindungen der Formel (II) kann beispielsweise 4:1 bis 1:4 betragen, bevorzugt betragen die molaren Verhältnisse 1:1 bis 1:4, besonders bevorzugt betragen die molaren Verhältnisse von Verbindungen der Formel (I) zu Verbindungen der Formel (II) im erfindungsgemäßen Verfahren 1:1 bis 1:2.

Bevorzugt weist (S)-3-N-Methylamino-1-(2-thienyl)-1-propanol eine Enantiomerenreinheit von 90 % oder mehr, besonders bevorzugt von 95 % oder mehr und ganz besonders bevorzugt von 99 % oder mehr auf.

Die Verbindungen p-Cymoldichlororutherium-Dimer und 5,5-N-p Tolnolyulfonyl-1,2-diphenylethylendiamin reagieren zusammen zum Katalysator. Deshalb ist von der Erfindung ebenfalls ein Verfahren umfasst, bei dem der Katalysator in Form der Verbindung [N-[(1S,2S)-2-(amino-κN)-1,2-diphenylethyl]-p-tolylsulfonamidato-κN]chloro[(η⁶)-p-cymol]-ruthenium(II) eingesetzt wird.

Das erfindungsgemäße Verfahren kann bei vermindertem, normalem oder erhöhtem Druck durchgeführt werden. Vorzugsweise arbeitet man bei Normaldruck oder erhöhtem Druck, z.B. bei 1 bis 16 bar, besonders bevorzugt bei 1 bis 10 bar. Ganz besonders bevorzugt wird die Reaktion bei Normaldruck durchgeführt.

Die eingesetzten Edukte oder/und Reaktanden sind teilweise käuflich zu erwerben oder sind nach dem Fachmann bekannten Verfahren synthetisierbar. So lässt sich z.B. 3-N-Methylamino-1-(2-thienyl)-1-propanon ausgehend von Acetylthiophen, Methylamin und Formaldehyd über eine Mannich-Reaktion (Mannich et al. Chem. Ber. 1922, 55, 356, Blicke et al. und J. Am. Chem. Soc. 1942, 64, 451) herstellen.

3-N-Methylamino-1-(2-thienyl)-1-propanon wird gemäß des erfindungsgemäßen Verfahrens als Verbindung oder/und in Form seiner Salze Methansulfonat oder Hydrochlorid eingesetzt.

Bevorzugt ist von der Erfindung ein Verfahren umfasst, bei dem 3-N-Methylamino-1-(2-thienyl)-1-propanon oder 3-N-Methylamino-1-(2-thienyl)-1-propanon Hydrochlorid oder 3-N-Methylamino-1-(2-thienyl)-1-propanon Methansulfonat, in Gegenwart von einem tertiären Amin, bevorzugt Triethylamin, in Gegenwart von Ameisensäure, in Gegenwart von p-Cymoldichlororuthenium-Dimer und in Gegenwart von (1S),(2S)-N-p-Toluolsulfonyl-1,2-diphenylethylendiamin zu (*S*)-3-N-Methylamino-1-(2-thienyl)-1-propanol umgesetzt wird.

Das erfindungsgemäße Verfahren wird vorzugsweise so durchgeführt, dass zunächst das Lösungsmittel und die Verbindungen der Formel (I) sowie die Verbindungen der Formel (II) vorgelegt werden. Anschließend wird, vorzugsweise unter Schutzgas, 3-N-Methylamino-1-(2-thienyl)-1-propanon oder/und dessen genannter Salze hinzugegeben und die Reaktionsmischung erwärmt. Vorzugsweise wird dann das Amin und die Ameisensäure oder/und das Formiat, vorzugsweise dosiert, hinzugegeben Die Reaktion kann durch Entfernen des entstehende Kohlendioxid, welches während der Reaktion freigesetzt wird, beschleunigt werden. Das Ende der Reaktion kann beispielsweise mittels HPLC bestimmt werden. Das Reaktionsgemisch kann zudem nach dem Fachmann bekannten Methoden, z.B. mittels Extraktion oder/und Destillation, aufgearbeitet werden.

Durch das erfindungsgemäße Verfahren kann (*S*)-3-N-Methylamino-1-(2-thienyl)-1-propanol in guten Ausbeuten und in hohen enantiomeren Reinheiten bis zu 99,9% hergestellt werden. Das erfindungsgemäße Verfahren eröffnet erstmals die Möglichkeit einer effizienten Herstellung von (*S*)-3-N-Methylamino-1-(2-thienyl)-1-propanol über eine asymmetrische Transferhydrierung in technischen Prozessen.

Die folgenden Beispiele dienen der beispielhaften Erläuterung der Erfindung und sind nicht als Beschränkung aufzufassen.

### Beispiele

### 1. Herstellung von (S)-3-N-Methylamino-1-(2-thienyl)-1-propanol ausgehend von 3-N-Methylamino-1-(2-thienyl)-1-propanon Hydrochlorid

77,5 g (0,76 mol) Triethylamin wurden bei 5 °C vorgelegt. Anschließend wurden 18,5 g (0,40 mol) Ameisensäure temperaturkontrolliert zudosiert, so dass die Temperatur 20 °C nicht überstieg. 90,0 g (84%ige Ware, 0,37 mol) 3-N-Methylamino-1-(2-thienyl)-1-propanon Hydrochlorid wurden zugegeben und die Reaktionsmischung mit 187 g Acetonitril verdünnt. Zur Reaktionsmischung wurde anschließend 2,0 g (22,9 mmol) Dimethylacetamid, 1,36 g (3,72 mmol) (S,S)-Ts-DPEN, 1,14 g (1,86 mmol) [(p-Cymol)RuCl₂]₂ und 42,5 mg (0,42 mmol) Triethylamin zugegeben. Die Reaktionssuspension wurde anschließend auf 35-37 °C erwärmt und über Nacht bei dieser Temperatur gerührt. Man erhielt 299,2 g einer 10,03gewichtsprozentigen Lösung von (S)-3-N-Methylamino-1-(2-thienyl)-1-propanol (48%) in Acetonitril mit einer Enantiomerenreinheit von >99,9%ee.

### 2. Herstellung von (S)-3-N-Methylamino-1-(2-thienyl)-1-propanol ausgehend von 3-N-Methylamino-1-(2-thienyl)-1-propanon Methansulfonat

260 g Methanol wurden vorgelegt und 0,456 g (1,25 mmol) (S;S)-Ts-DPEN und 0,381 g (0,62 mmol) [(p-Cymol)RuCl₂]₂ zugegeben und die Reaktionsmischung für 1 h bei 40 °C gerührt. 53,9 g (0,15 mol) 3-N-Methylamino-1-(2-thienyl)-1-propanon Methansulfonat wurden zugegeben und eine Mischung aus 9,2 g Triethylamin (0,09 mol) und 22,9 g Ameisensäure (0,50 mol) wurden zudosiert. Dabei ging der Feststoff in Lösung. Die Reaktionslösung wurde auf 45 °C erwärmt und 4 h bei 45 °C gerührt. 0,456 g (1,25 mmol) (S;S)-Ts-DPEN und 0,381 g (0,62 mmol) [(p-Cymol)RuCl₂]₂ wurden erneut zugegeben und die Reaktionslösung für weitere 2 h gerührt. Man erhielt 278,5 g einer 3,36gewichtsprozentigen Lösung von (S)-3-N-Methylamino-1-(2-thienyl)-1-propanol (48%) in Acetonitril mit einer Enantiomerenreinheit von >99,9%ee.

### 3. Herstellung von (S)-3-N-Methylamino-1-(2-thienyl)-1-propanol ausgehend von 3-N-Methylamino-1-(2-thienyl)-1-propanon Methansulfonat mit S,S-DPEN als Ligand (Vergleichsbeispiel)

In einem 100 mL Rundkolben mit Magnetrührer, Liebigkühler und Stickstoffanschluss wurden 36,6 g Methanol, 77.7 mg (0,13 mmol) [(p-Cymol)RuCl₂]₂ und 67,3 mg (0,31 mmol) S,S-DPEN vorgelegt. Anschließend wurden 5.00 g 3-N-Methylamino-1-(2-thienyl)-1-propanon Methansulfonat zugegeben und die Reaktionsmischung auf 40 °C erwärmt. Bei 40-45 °C wurden anschließend 1,2 g Triethylamin sowie 3,0 g Ameisensäure zugegeben und die Reaktionslösung bei 45 °C gerührt. Umsatzproben wurden nach 23 Stunden und 47 Stunden gezogen. Dabei entstand kein Umsatz zum (S)-3-N-Methylamino-1-(2-thienyl)-1-propanol.

### 4. Herstellung von (S)-3-N-Methytamino-1-(2-thienyl)-1-propanol ausgehend von 3-N-Methylamino-1-(2-thienyl)-1-propanon Methansulfonat mit S-Tol-BINAP als Ligand (Vergleichsbeispiel)

In einem 100 mL Rundkolben mit Magnetrührer, Liebigkühler und Stickstoffanschluss wurden 36,6 g Methanol, 77.7 mg (0,13 mmol) [(p-Cymol)RuCl₂]₂ und 215 mg S-Tol-BINAP vorgelegt. Anschließend wurden 5.00 g 3-N-Methylamino-1-(2-thienyl)-1-propanon Methansulfonat zugegeben und die Reaktionsmischung auf 40 °C erwärmt. Bei 40-45 °C wurden anschließend 1,2 g Triethylamin sowie 3,0 g Ameisensäure zugegeben und die Reaktionslösung bei 45 °C gerührt. Umsatzproben wurden nach 23 Stunden und 47 Stunden gezogen. Nach 47 Stunden wurde ein Umsatz von lediglich 0,4% erreicht.

### 5. Herstellung von (S)-3-N-Methylamino-1-(2-thienyl)-1-propanol ausgehend von 3-N-Methylamino-1-(2-thienyl)-1-propanon Methansulfonat mit S,S-Ts-DPEN als Ligand

In einem 100 mL Rundkolben mit Magnetrührer, Liebigkühler und Stickstoffanschluss wurden 36,6 g Methanol, 77.7 mg (0,13 mmol) [(p-Cymol)RuCl₂]₂ und 93 mg (0,25 mmol) S,S-Ts-DPEN vorgelegt. Anschließend wurden 5.00 g 3-N-Methylamino-1-(2-thienyl)-1-propanon Methansulfonat zugegeben und die Reaktionsmischung auf 40 °C erwärmt. Bei 40-45 °C wurden anschließend 1,2 g Triethylamin sowie 3,0 g Ameisensäure zugegeben und die Reaktionslösung bei 45 °C gerührt. Umsatzproben wurden nach 23 Stunden und 47 Stunden gezogen. Bereits nach 23 h wurde ein Umsatz von 80% erreicht. Nach 47 Stunden betrug der Umsatz 83%.

## Patentansprüche

1. Verfahren zur Herstellung von (*S*)-3-N-Methylamino-1-(2-thienyl)-1-propanol und dessen Salze, **dadurch gekennzeichnet, dass** 3-N-Methylamino-1-(2-thienyl)-1-propanon - hydrochlorid oder-methansulfat in Gegenwart von p-Cymoldichlororuthenium-Dimer und S,S-N-p-Toluol-sulfonyl-1,2-diphenylethylendiamin sowie in Gegenwart mindestens eines Amins und in Gegenwart von Ameisensäure oder/und Formiaten oder Mischungen davon umgesetzt wird.

2. Verfahren nach Anspruch 1, **dadurch gekennzeichnet, dass** das Amin ein tertiäres Amin, bevorzugt Triethylamin, darstellt.

3. Verfahren nach einem oder mehreren der Ansprüche 1 bis 2, **dadurch gekennzeichnet, dass** das Verfahren in Gegenwart eines Lösungsmittels ausgewählt aus der Gruppe Amide, Nitrile oder Alkohole oder Mischungen dieser Lösungsmittel durchgeführt wird.

4. Verfahren nach einem oder mehreren der Ansprüche 1 bis 3, **dadurch gekennzeichnet**, das das molare Verhältnis von Ameisensäure zu tertiärem Amin zwischen 3:1 bis 0,8:1 beträgt.

5. Verfahren nach einem oder mehreren der Ansprüche 1 bis 4, **dadurch gekennzeichnet, dass** das Verfahren bei einer Temperatur von 30°C bis 60 °C durchgeführt wird.

6. Verfahren nach einem oder mehreren der Ansprüche 1 bis 5, **dadurch gekennzeichnet, dass** die eingesetzte molare Menge an Ruthenium im p-Cymoldichlororuthenium bezogen auf die Menge von 3-N-Alkylamino-1-(2-thienyl)-1-propanon- hydrochlorid oder -methansulfat zwischen 0,05 bis 1 mol-% beträgt.

7. Verfahren nach einem oder mehreren der Ansprüche 1 bis 6, **dadurch gekennzeichnet, dass** (*S*)-3-N-Methylamino-1-(2-thienyl)-1-propanol in einer Enantiomerenreinheit von mehr als 90 % hergestellt wird.

## Claims

1. Method for preparing (*S*)-3-N-methylamino-1-(2-thienyl)-1-propanol and salts thereof, **characterized in that** 3-N-methylamino-7-(2-thienyl)-1-propanone hydrochloride or methanesulphonate is reacted in the presence of p-cymenedichlororuthenium dimer and S,S-N-p-toluenesulphonyl-1,2-diphenylethylenediamine and also in the presence of at least one amine and in the presence of formic acid or/and formates or mixtures thereof.

2. Method according to Claim 1, **characterized in that** the amine is a tertiary amine, preferably triethylamine.

3. Method according to one or more of Claims 1 and 2, **characterized in that** the method is carried out in the presence of a solvent selected from the group consisting of amides, nitriles or alcohols or mixtures of these solvents.

4. Method according to one or more of Claims 1 to 3, **characterized in that** the molar ratio of formic acid to tertiary amine is from 3:1 to 0.8:1.

5. Method according to one or more of Claims 1 to 4, **characterized in that** the method is carried out at a temperature of 30°C to 60°C.

6. Method according to one or more of Claims 1 to 5, **characterized in that** the molar amount of ruthenium used in the p-cymenedichlororuthenium, based on the amount of 3-N-alkylamino-1-(2-thienyl)-1-propanone hydrochloride methanesulphonate, is from 0.05 to 1 mol%.

7. Method according to one or more of Claims 1 to 6, **characterized in that** (*S*)-3-N-methylamino-1-(2-thienyl)-1-propanol is prepared with an enantiomeric purity of more than 90 %.

## Revendications

1. Procédé de préparation de (S)-3-N-méthylamino-1-(2-thiényl)-1-propanol et ses sels, **caractérisé en ce que** du chlorhydrate ou du méthanesulfonate de 3-N-méthylamino-1-(2-thiényl)-1-propanone est mis en réaction en présence de dimère de p-cymène-dichlororuthénium et de S,S-N-p-toluène-sulfonyl-1,2-diphényléthylènediamine, ainsi qu'en présence d'au moins une amine et en présence d'acide formique et/ou de formiates ou leurs mélanges.

2. Procédé selon la revendication 1, **caractérisé en ce que** l'amine est une amine tertiaire, de préférence la triéthylamine.

3. Procédé selon une ou plusieurs des revendications 1 à 2, **caractérisé en ce que** le procédé est réalisé en présence d'un solvant choisi dans le groupe constitué par les amides, les nitriles ou les alcools ou les mélanges de ces solvants.

4. Procédé selon une ou plusieurs des revendications 1 à 3, **caractérisé en ce que** le rapport molaire entre l'acide formique et l'amine tertiaire est compris entre 3:1 et 0,8:1.

5. Procédé selon une ou plusieurs des revendications 1 à 4, **caractérisé en ce que** le procédé est réalisé à une température de 30 °C à 60 °C.

6. Procédé selon une ou plusieurs des revendications 1 à 5, **caractérisé en ce que** la quantité molaire utilisée de ruthénium dans le p-cymène-dichlororuthénium par rapport à la quantité de chlorhydrate ou de méthanesulfonate de 3-N-alkylamino-1-(2-thiényl)-1-propanone est comprise entre 0,05 et 1 % en moles.

7. Procédé selon une ou plusieurs des revendications 1 à 6, **caractérisé en ce que** le (S)-3-N-méthylamino-1-(2-thiényl)-1-propanol est préparé en une pureté énantiomérique supérieure à 90 %.
